# FASCICULE DE BREVET EUROPEEN

(11) **EP 1 223 999 B1**
(45) Date de publication et mention de la délivrance du brevet: **31.05.2006**
(21) Numéro de dépôt: 00967987.9
(22) Date de dépôt: 10.10.2000
(51) Int. Cl.: A61M 5/315, A61M 5/28

(54) **DISPOSITIF POUR RECONSTITUER UNE SOLUTION, UNE SUSPENSION OU UNE DISPERSION THERAPEUTIQUE**
VORRICHTUNG ZUR WIEDERHERSTELLUNG EINER THERAPEUTISCHEN SUSPENSION ODER DISPERSION
DEVICE FOR RECONSTITUTING A THERAPEUTIC SOLUTION, SUSPENSION OR DISPERSION

(30) Priorité: 13.10.1999 FR 9912748
(43) Date de publication de la demande: 24.07.2002
(73) Titulaire: SOCIETE DE CONSEILS DE RECHERCHES ET D'APPLICATIONS SCIENTIFIQUES (S.C.R.A.S.), 75016 Paris (FR)
(72) Inventeur: CHERIF-CHEIKH, Roland, 08860 Castelldefels (ES); AUBERT, Christophe, 08810 Les Roquettes (ES)
(74) Mandataire: Bourgouin, André
(86) Numéro de dépôt international: PCT/FR2000/002804
(87) Numéro de publication internationale: WO 2001/026718

(56) Documents cités:
- EP-A- 0 298 585
- WO-A-95/32015
- FR-A- 2 604 363
- US-A- 4 581 016
- US-A- 5 395 325

## Description

La présente invention a pour objet un dispositif pour la préparation extemporanée d'une solution, d'une suspension ou d'une dispersion, par exemple de formulations injectables préférentiellement selon un procédé de reconstitution sous l'action du vide dit VAC *("Vacuum Automatic Control")* faisant l'objet d'une demande de brevet français antérieure n° 96/06886. La présente invention a aussi pour objet un procédé de préparation et de conditionnement desdites formulations dans lesdits dispositifs selon lequel on fermera ensuite le réservoir desdits dispositifs contenant la partie solide de ladite formulation par le côté du piston, opposé à son injection ou à sa libération après reconstitution.

Selon l'invention, le dispositif est préférentiellement utilisé comme dispositif dans lequel une partie de la formulation est conditionnée sous vide et une autre est constituée de la partie liquide de la préparation finale, chacune d'elles étant conditionnée dans un réservoir distinct formant partie dudit dispositif.

Dans les préparations thérapeutiques et, à titre d'exemple, plus particulièrement dans le cas des formes injectables, il est parfois utile, préférable voire indispensable de séparer les constituants. Le dispositif et le procédé faisant l'objet de la présente invention s'intéressent plus particulièrement aux cas dans lesquels une partie desdits constituants est sous une forme non liquide et une autre sous une forme liquide. Notamment, ils pourront être appliqués à tout lyophilisat de solutions injectables, à une poudre ou toutes autres formes solides conditionnées sous vide telles qu'elles puissent former, après reconstitution, un liquide, un gel ou une forme pâteuse injectable.

A titre d'exemple d'une forme illustrant l'intérêt de cette invention, et sans que cela soit limitatif vis-à-vis des autres applications possibles, nous pouvons retenir les formes injectables dans lesquelles une partie est sous forme sèche ou solide et une autre constitue le véhicule liquide d'injection.

Plus précisément, la partie solide contiendra en général le principe actif et pourra être une forme lyophilisée et le véhicule d'injection pourra être une préparation aqueuse destinée à hydrater la forme sèche avant injection. L'objectif est alors de reconstituer une solution ou une suspension voire une dispersion injectable.

Ce type de forme injectable est largement répandu et les raisons pour lesquelles on est amené à les utiliser plutôt qu'une forme liquide mélangée sont bien connues.

On peut citer notamment les problèmes de stabilité ou compatibilité des constituants dans le mélange liquide. Si un tel conditionnement séparé solide-liquide permet d'améliorer certains points comme par exemple la conservation de la formulation dans le temps à une température compatible avec son acheminement et son stockage, il n'en reste pas moins que c'est un arrangement qui pose de nombreux problèmes spécifiques notamment liés à l'existence des deux sous-ensembles.

La solution traditionnelle pour ce type de préparation est le conditionnement en flacon de la forme solide ou lyophilisée, qui est hydratée au moment de l'emploi par transfert depuis une seringue d'injection du milieu liquide à l'aide d'une aiguille piquée à travers le bouchon du flacon. Une fois hydratée, la formulation est généralement récupérée par la même aiguille dans ladite seringue. Cette seringue pourra avoir été préalablement chargée du liquide à partir d'une ampoule ou d'un premier flacon ou bien il pourra s'agir d'une seringue pré-remplie de la phase liquide du mélange.

Cette opération relativement compliquée met plusieurs fois en mouvement les éléments constitutifs de la formulation et présente des risques de contamination (ou de contact avec l'aiguille) autant au niveau de l'utilisateur que du patient receveur. La variabilité des résultats et donc du traitement administré reste non négligeable en fonction de paramètres impondérables comme l'habileté ou la pratique de l'opérateur.

Un certain nombre de solutions techniques au niveau du dispositif ont donc été développées pour essayer de simplifier cette préparation et/ou de diminuer les risques.

Certaines solutions restent assez proches du procédé standard à partir d'un conditionnement en plusieurs éléments et cherchent seulement à réduire une partie du risque ou du problème. C'est notamment le cas des solutions techniques dans lesquelles un élément intermédiaire est ajouté entre le flacon et la seringue pour simplifier l'injection et la récupération du liquide. On peut, par exemple, citer le dispositif Monovial® de Becton Dickinson ou Vial-Mate® de Baxter.

Si cette approche ne simplifie guère la préparation extemporanée et ne rentre pas dans la catégorie des conditionnements ou seringues pré-remplis, elle a surtout aux yeux de l'industrie pharmaceutique le mérite de ne pas modifier le conditionnement primaire en flacon de la forme solide synonyme d'un procédé de remplissage et de traitement parfaitement validé et connu, faisant appel à des équipements existants.

Il existe, bien sûr, d'autres approches du dispositif plus radicalement novatrices pour simplifier ce problème qui fait en général appel à un conditionnement ou seringue pré-remplie. On peut, par exemple, citer l'utilisation des seringues bi-compartimentales généralement équipées de déviations ou by-pass au niveau desquels pourront se désactiver les séparations mobiles existant entre les deux parties d'un même réservoir. A titre d'exemple, on peut citer les seringues Hypack liqui-dry® de Becton Dickinson ou la seringue Lyoject® de Vetter. Dans ce cas, la séparation mobile est un piston qui, au moment de la réhydratation, se déplacera en regard d'une déviation pratiquée dans la paroi de la seringue.

Cette solution élégante présente l'avantage de réduire les éléments de l'injectable à un seul dispositif unitaire. Dans sa version basique, cette solution peut présenter des risques d'erreur ou de fausses manipulations lors de l'opération de reconstitution ; toutefois certaines améliorations pratiquées peuvent permettre de réduire significativement ces risques.

L'inconvénient majeur pour l'industrie pharmaceutique d'un tel dispositif reste lié au conditionnement dans un seul et même réservoir de deux constituants dont l'un est liquide et l'autre solide et qui n'ont habituellement pas du tout le même mode de traitement et/ou de stérilisation surtout lorsque le solide est préparé par lyophilisation. Un autre inconvénient dans ce cas est lié à la taille ou volume occupé par la bi-compartimentale dans le lyophilisateur qui, étant donné la partie du réservoir ensuite prévue pour la partie liquide, pourra être deux fois plus importante que dans les réservoirs séparés.

A cela s'ajoute le risque de stabilité lié à la proximité et possibilité de contact entre les deux parties du réservoir à travers la séparation mobile ou piston.

Face à ce problème, d'autres approches du conditionnement en un seul dispositif ont été tentées dans lesquelles le conditionnement est, cette fois-ci, réalisé en deux réservoirs bien distincts.

A titre d'exemple, dans ce sens, on pourra citer la demande de brevet PCT WO 94/13344 (Meyer), qui décrit un dispositif dans lequel le solvant ou liquide est conditionné dans une carpule qui peut être standard et la seconde partie de la formulation (qui peut être un lyophilisat) est contenue dans un tube spécifique droit à fond fermé.

On peut aussi citer la demande de brevet EP 298 585 qui décrit une seringue constituée de plusieurs éléments emboîtables incluant deux réservoirs fermés avant utilisation et susceptibles d'être mis en communication par une aiguille à double pointe.

Les pistons dits "obturateurs-pistons-vannes" sont particuliers et notamment le piston du tube qui comporte des rainures pour un pré-positionnement en lyophilisation, synonyme également de volume mort.

Le dispositif est conçu pour être entièrement assemblé, ce qui implique de réaliser cette opération en aseptique ou d'envisager une post-stérilisation commune liquide-solide qui risque d'être incompatible avec l'une des phases ou avec l'orientation actuelle de certaines directives. En cela, ce dispositif ne résout pas le problème de conditionnement, et donc de traitement, unitaire des bi-compartimentales.

En fonction du très large développement industriel de certains procédés de remplissage autour de réservoirs ou contenus standards et des nécessités connues de préparation aseptique ou de post-stérilisation usuelle, il serait hautement souhaitable de pouvoir, dans ce cas précis de formulation en deux sous-unités, disposer d'un dispositif simplifiant et sécurisant la préparation extemporanée tout en permettant l'utilisation de traitements standards de sous-ensembles réservoirs existants. Ce besoin implique de préférence l'utilisation d'un dispositif pré-rempli évitant les transferts problématiques. Pour certains produits très fragiles ou sophistiqués, il est important d'éviter l'agitation et les déplacements créés par ces transferts.

Un autre aspect du problème lié aux opérations de mélange des deux parties de la formulation puis de dégazage (avec risque de contamination en fonction de l'importance et du temps de contact avec le milieu ambiant) explique la recherche de dispositifs dans lesquels ce mélange est simplifié et, si possible, réalisé sans ouverture préalable sur l'extérieur, voire de dispositifs dans lesquels le dégazage pourrait être évité.

La façon dont la reconstitution est réalisée et notamment sa vitesse de réalisation et les pertes liées au transfert et au dégazage restent, quel que soit le dispositif, des paramètres aléatoires pouvant entraîner des résultats dissemblables.

La demande de brevet français n° 96/06886 décrit un procédé selon lequel cette reconstitution devient automatique après activation sous l'action du vide et dans lequel l'opération de dégazage est tout simplement supprimée.

La demanderesse a maintenant inventé des dispositifs avantageux qui satisfont à l'ensemble de ces *desiderata,* par exemple sous la forme d'un arrangement "en seringue" très proche de celui communément connu qui permet de conserver les mêmes gestes thérapeutiques et d'utiliser les réservoirs les plus classiques. Ces dispositifs et ces arrangements sont caractérisés en ce que la partie non liquide de la formulation à injecter est conditionnée dans le corps de la seringue et la partie liquide de reconstitution est conditionnée à part dans un réservoir qui peut former la tige d'injection du piston de la seringue. Ces arrangements sont réalisés, notamment à partir de réservoirs classiques, en appliquant le procédé de préparation et conditionnement qui constitue l'autre partie de l'invention et qui consiste à remplir et fermer par l'arrière au niveau du piston.

De même, le système de transfert entre les deux parties est assuré par une aiguille, un tube ou une tige qui présente la particularité de pénétrer dans le réservoir non liquide à travers le piston qui va servir à l'injection de la forme reconstituée.

Ces dispositifs, selon l'invention, présentent de plus un moyen de blocage dudit piston d'injection qui permet l'implantation dudit système de transfert dans ledit piston ; ledit moyen de blocage pourra ensuite être désactivé, libérant ainsi ledit piston d'injection.

Enfin, le dispositif peut présenter un mécanisme par lequel, après pénétration dans ledit piston, ledit système de transfert est retiré avant l'injection pour retrouver l'étanchéité du réservoir contenant la formulation à injecter.

Ce retrait sera, par exemple, selon le dispositif détaillé par la suite, combiné avec le déblocage du piston d'injection.

Un certain nombre d'autres applications plus précises, dérivées ou corollaires des dispositifs selon l'invention, seront précisées dans les descriptions plus détaillées qui suivent.

Les dispositifs et procédés objets de la présente invention peuvent fonctionner de façon avantageuse en utilisant un procédé de reconstitution qui permet directement une injection sans dégazage préalable. Une fois reconstituée, la formulation est immédiatement utilisable à la pression atmosphérique sans risque de surpression (ou de dépression) ; aucun risque, donc, de nébulisation ou de jet accidentel du produit au moment de sa connexion avec l'aiguille d'injection. Ceci, associé à l'absence de dégazage, élimine d'important soucis comme la contamination au contact du milieu ambiant et le risque de piqûres accidentelles, l'aiguille n'ayant plus à être manipulée à l'air libre avant implantation. On pourra même avantageusement connecter cette aiguille à la formulation reconstituée seulement après l'avoir introduit dans le corps, ce qui permet de pratiquer le test de veine par simple visualisation de l'autre extrémité de l'aiguille après implantation.

Un des réservoirs les plus classiques correspondant aux nécessités de standardisation existantes est la carpule ou cartouche d'injection, terminée à une extrémité par un piston classique et à l'autre par un col fermé, par un opercule ou septum ou par un bouchon et sertie par une capsule métallique.

De même, les dispositifs selon l'invention peuvent utiliser de façon avantageuse de telles carpules standard pour leurs deux réservoirs ainsi que les pistons et bouchons existants. Les conditions de remplissage et de traitement de stérilité pour leurs deux réservoirs pourront être exactement les mêmes que celles généralement appliquées à ce type de conteneur.

Ainsi le dispositif selon l'invention apporte-t-il une solution nouvelle et simple qui répond entre autres aux questions, aux problèmes et aux besoins précédemment évoqués.

De plus, l'utilisation du système de réhydratation automatique par le vide constitue en soi un contrôle d'intégrité direct de l'emballage primaire contenant le principe actif, à savoir le réservoir ou carpule. Son fonctionnement VAC est un gage unique, que l'asepsie du réservoir a bien été préservée pendant le stockage. Ce contrôle pourra être associé à l'utilisation d'un emballage secondaire sous vide qui assurera le même contrôle pour l'ensemble du dispositif et permettra éventuellement de relayer le contrôle du vide sur la durée du stockage.

Les descriptions qui suivent (générales ou plus précises à partir des figures) ne constituent que quelques exemples avantageux de modes de réalisation du dispositif selon l'invention, lequel pourra être mis en oeuvre selon d'autres spécificités également couvertes par l'invention. En plus d'un dispositif possible détaillé, certaines autres alternatives seront ainsi présentées.

Ce dispositif, selon l'invention, pourra s'appliquer moyennant quelques modifications mineures mais toujours selon les mêmes principes mécaniques à de nombreuses variations dans le domaine de l'injectable comme dans d'autres domaines thérapeutiques. Certaines précisions concernant ces autres applications possibles telles que, par exemple l'utilisation en stylo-injecteur, seront données en fonction de leur relation avec la matière inventive.

Dans l'exemple décrit et selon un mode avantageux de l'invention, le dispositif est présenté avec une séparation totale des deux réservoirs, à savoir celui recelant les éléments non liquides (ici lyophilisat) et celui recelant les éléments liquides (ici milieu aqueux de remise en suspension). Autrement dit, il n'existe aucune relation par élément physique entre les deux réservoirs avant utilisation.

Lesdits réservoirs pourront d'ailleurs avantageusement être emballés de façon séparée et individuelle. Toutefois, comme cela sera précisé, il est possible d'envisager le dispositif dans un pré-arrangement dans lequel les deux sous-ensembles sont associés voire inclus dans le même emballage.

L'aspect procédé de l'invention concerne les spécificités de la préparation et du conditionnement liés à l'utilisation de réservoirs standard et que permet l'utilisation du dispositif, notamment autour de la lyophilisation.

Même si le dispositif de l'invention pourrait être réalisé avec d'autres types de réservoir et notamment avec des tubes en verre, dans la forme de réalisation avantageuse du dispositif décrit en détail, on utilisera donc, comme réservoirs, deux carpules standards, par exemple de capacités 2,25 et 3,15 ml.

La carpule contenant la partie non liquide, ici le lyophilisat, constitue le réservoir proximal situé à l'intérieur dudit dispositif et directement en contact avec la zone de sortie de l'aiguille d'injection. C'est dans ce réservoir proximal, qui en général contient le principe actif de la préparation, que se reconstitue la formulation à injecter qui se trouve donc ensuite être, en terme d'arrangement, dans un mode équivalent à l'utilisation traditionnelle des carpules d'injection, exactement en regard de l'aiguille double pointe.

La carpule contenant la partie liquide ou milieu aqueux d'injection constitue le réservoir distal du dispositif. Elle est située à l'autre extrémité du dispositif d'injection et sert également de tige de piston lors de l'injection de la préparation par la seringue qu'elle forme avec l'ensemble. Elle pénètre donc plus ou moins profondément à l'intérieur du corps du dispositif seringue et à l'intérieur de la carpule proximale.

Entre ces deux carpules se trouvent les différentes pièces ou éléments du mécanisme de connexion, qui peuvent être fixés sur le piston de la carpule proximale. L'ensemble est préférablement arrangé à l'intérieur d'un corps plastique qui guide ou contrôle certaines des fonctions et qui protège les réservoirs en verre.

Cet élément extérieur qui constitue le corps de la seringue d'injection que forme le dispositif, sera préférentiellement de forme cylindrique, pour sa partie inférieure et comportera l'appuie-doigt de ladite seringue dans sa partie supérieure. Chaque partie pourra être un sous-élément distinct assemblé à l'autre au moment du montage. La partie inférieure de ce corps cylindrique pourra avoir, de plus, une fonction de support et de guidage de la carpule et du piston d'injection lors des opérations de lyophilisation et de conditionnement sous vide.

L'extrémité proximale de ladite partie inférieure supportera l'aiguille d'injection qui pourra être préfixée comme représentée plus loin ou bien, plus classiquement, montée sur une embase qui viendra se fixer sur ladite extrémité proximale. Avantageusement, cette extrémité pourra donc constituer un troisième élément extérieur fixé sur la partie inférieure.

Le dispositif est donc présenté par la suite avec une solution spécifique d'aiguille pré-montée et de capuchon-verrou qui évite le risque d'activation avant reconstitution de la formulation. Il est évidemment possible d'utiliser ce dispositif avec toute autre aiguille et notamment les aiguilles non pré-montées, par exemple celles du type classique utilisées sur les injecteurs ou les stylos à carpules.

De même, en fonction d'autres applications (perfusion, ophtalmologie, etc.), le dispositif pourrait être associé à un connecteur ou aiguille de transfert, un distributeur de goutte ou un spray.

Du côté distal, la solution détaillée garde la carpule de liquide comme tige de piston. Dans le cas, par exemple, d'un stylo-injecteur, l'ensemble du corps du dispositif peut constituer la partie proximale de l'injecteur et il est possible d'envisager de retirer la carpule distale après reconstitution et de la remplacer par le mécanisme de dosage dudit stylo, qui viendrait refermer le corps du dispositif.

Cette solution est particulièrement avantageuse étant donné la précision de la dose totale reconstituée et le faible volume dans le stylo-injecteur, sans carpule distale ni carpule bi-compartimentale, que rend possible l'utilisation du dispositif selon le procédé VAC, permettant ainsi de ramener le cas d'un lyophilisat au cas simple et classique d'une solution.

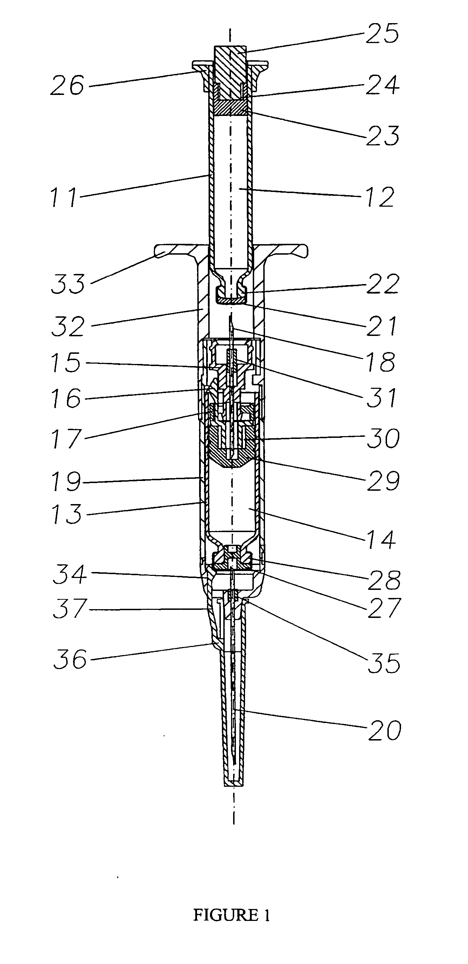

Pour des raisons de clarté et d'enchaînement logique des opérations dans un ordre chronologique, après la figure 1 générale du dispositif détaillé selon l'invention avec les figures qui suivent, nous décrirons, dans un premier temps, les procédés de remplissage et conditionnement (dont certains aspects constituent la seconde partie, c'est-à-dire le procédé de préparation de l'invention), avant de préciser le dispositif de l'invention qui en est issu. Ces procédés correspondent au montage du dispositif de l'invention qui en est issu et permettent de préciser ledit dispositif et les fonctions des éléments qui le constituent. Nous finirons, enfin, dans les figures, par la description de l'utilisation dudit dispositif.

Le dispositif précis et spécifique représenté par les figures suivantes ne constitue qu'une solution possible de réalisation du dispositif et du procédé selon l'invention, qui n'entend en aucun cas limiter la portée générale de ladite invention.

Par exemple, les éléments dits de verrou représentés par la suite pourraient être remplacés par un système de clé ou goupille latérale susceptible de bloquer le piston de la carpule conditionnée sous vide non seulement sur son embase en verre mais également sur le fourreau plastique qui l'entoure. Ladite clé pourrait être déverrouillée (ou dégoupillée) après reconstitution selon le procédé VAC. Pour des raisons de commodité et d'espace dans le lyophilisateur, ledit système de clé pourrait être muni d'une prise en main ou poignée d'activation amovible ou indépendante.

De même, même si un des avantages des dispositifs, selon l'invention, en terme de remplissage, stérilisation et conditionnement, peut être la séparation physique des deux réservoirs, il est tout à fait possible de les assembler préalablement, soit après stérilisation (en aseptique), soit avant stérilisation (avec une post-stérilisation de l'ensemble). Dans le cas de la solution assemblée, la carpule de liquide peut être munie d'un dispositif de blocage/déblocage dans le corps de la seringue qui empêche sa manipulation accidentelle. Elle peut également assurer (par exemple par un joint) la fermeture aseptique du corps de la seringue.

Dans la solution représentée par les figures, il est également possible de prévoir un dispositif ou élément qui protège la membrane ou le septum de la carpule soit du type *flip-off,* soit encore un système, par exemple rétractable sur le tube, qui va protéger ce septum jusqu'à l'introduction du col dans le corps de seringue. De même, du côté de ce corps et par exemple au niveau de l'appuie-doigt, il est possible de prévoir une protection, amovible ou perçable par exemple, qui protège l'asepsie de l'aiguille de transfert.

Enfin, le dispositif est présenté dans une solution aiguille pré-montée. Il est possible de concevoir le dispositif, selon l'invention, simplement équipé d'un bouchon du côté proximal de la tête de la carpule ; bouchon qui sera retiré après reconstitution du mélange, et remplacé par une aiguille dont le montage par son embase pourra assurer le percement de la membrane ou bouchon de la carpule. Ce percement pourra être réalisé soit par une aiguille double pointe, soit par une pointe plastique, élément de l'embase dont le canal sera connecté à l'aiguille. Afin de préciser la partie générale de l'invention en terme de dispositif et de procédé de préparation et conditionnement, à la suite de l'exemple détaillé seront présentées d'autres possibilités et leur procédé de traitement sera résumé.

Une solution, adaptable au traitement en aseptique et aux volumes plus faibles, sera ainsi évoquée. Une solution simplifiée et adaptable aux petits volumes et aux stylo-injecteurs sera aussi décrite. Enfin, une solution alternative à la solution détaillée dans laquelle certains automatismes du mécanisme sont abandonnés au profit d'une simplification générale. A chaque fois, le procédé spécifique de l'invention sera précisé.

### Figures 1 à 13

La figure **1** représente une vue générale du dispositif de l'invention après installation de la tige de piston formée par la carpule de liquide et avant activation.

Les figures **2** et **3** schématisent le procédé de remplissage classique autour de la carpule standard contenant la phase liquide, puis son assemblage spécifique pour pouvoir être ensuite utilisée dans le dispositif de l'invention.

Les figures **4** à **6** illustrent les étapes de conditionnement de la phase solide de la formulation dans une carpule standard et dans le cas particulier d'une forme en dispersion dans un lyophilisat.

La figure **7** représente les étapes d'assemblage de la partie proximale du dispositif autour de la forme solide ou lyophilisée.

Les figures **8** et **9** décrivent, après association des deux sous-ensembles du dispositif (à savoir le corps de la seringue contenant la carpule de solide et la tige de la seringue qui est la carpule du liquide), la reconstitution VAC automatique et le mécanisme de fonctionnement du dispositif de l'invention.

La figure **10** représente l'injection dans l'organisme de la formulation reconstituée dans le dispositif de l'invention.

La figure **11** représente une alternative de dispositif autour d'un autre mécanisme de transfert utilisable en aseptique.

La figure **12** illustre un dispositif simplifié pour petits volumes et pour stylo-injecteurs.

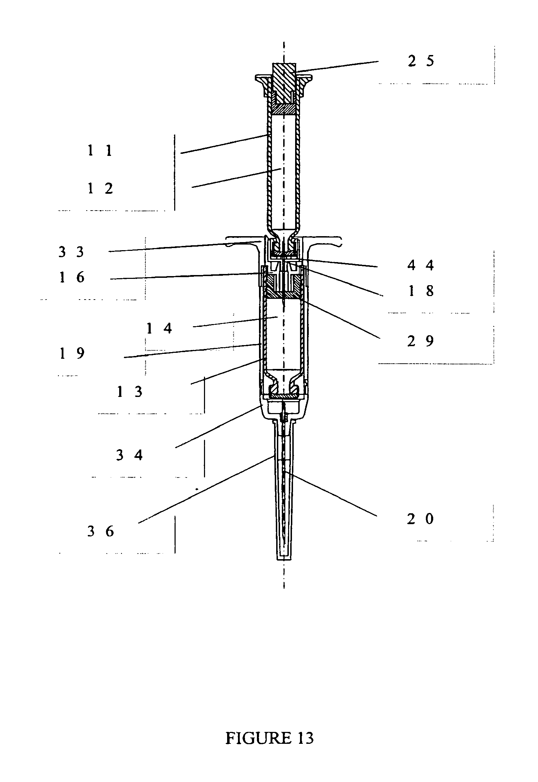

Enfin, la figure **13** représente l'application du dispositif simplifié au cas de la formulation et du volume du dispositif précédemment détaillé.

### Description détaillée des figures

La figure 1 représente une forme précise ou spécifique du dispositif permettant la réalisation d'une solution ou suspension médicinale, une solution de peptides ou protéines par exemple, ou une suspension injectable de microsphères PLGA permettant la libération prolongée d'un principe actif (formes retard), selon le procédé VAC, à partir de 2 éléments réservoirs de base, ici, des carpules. En particulier, la figure 1 représente ce dispositif avant reconstitution, une fois installée la carpule distale faisant office de tige de piston et reconstituant ledit dispositif sous une apparence très similaire à une seringue classique. Cette version correspond à la solution pour 2 ml maximum de volume à injecter ; le réservoir distal pourra être plus ou moins rempli et déterminer le volume reconstitué.

Ce dispositif seringue comporte un premier conteneur **11** constitué par la carpule dans lequel est stocké un solvant ou milieu aqueux 12, un second conteneur 13 constitué par la carpule dans lequel la forme solide ou lyophilisat **14** est conditionnée sous vide, 4 éléments de transfert, à savoir la clé supérieure **15,** le verrou **16,** la clé inférieure **17** et l'aiguille de transfert 18, et enfin un fourreau 19 dans lequel se trouve le réservoir 13 et où pénètre, à l'extrémité distale, le réservoir **11** faisant office de tige de piston et sur lequel est fixée, à l'extrémité proximale, l'aiguille d'injection 20. On notera que le lyophilisat **14** qui correspond dans l'exemple à une suspension de microsphères (forme retard) pourra être remplacé par tout lyophilisat de solutions injectables, par une poudre ou toutes autres formes solides conditionnées sous vide telles qu'elles puissent former, après reconstitution, un liquide, un gel ou une forme pâteuse utilisable dans ledit dispositif.

Le premier conteneur 11, formant réservoir ou carpule distale et tige de piston de la seringue, sera préférentiellement stocké et conditionné à part du reste de la seringue, par exemple dans un emballage individuel. Tout le reste du dispositif pourra avantageusement être conditionné ensemble dans un second emballage.

La carpule 11 du premier conteneur qui contient le milieu liquide **12** est fermée à ses deux extrémités de façon standard. A son extrémité proximale, le moyen de fermeture sera par exemple une membrane ou un disque de fermeture 21 fixé au col de la carpule par sertissage d'une capsule 22 percée en son centre et qui pourra, par exemple, être métallique. Cette solution équivalente à celle utilisée entre autre dans les carpules dentaires permettra notamment un remplissage par débordement évitant toutes bulles d'air à l'intérieur du liquide. A son extrémité distale, la carpule 11 est fermée par un piston 23 qui pourra être un piston classique d'injection, par exemple en caoutchouc ou en polybromobutyle. Ce piston comporte un pas de vis interne 24 où vient habituellement se fixer la tige de piston.

Dans le cas du dispositif, ce pas de vis 24 sert à fixer par vissage la courte tige de purge 25 qui activera la réhydratation par "décollage" du piston 23 et purgera l'aiguille de transfert 18, par son remplissage, après percement de la membrane 21. La longueur de ladite tige sera fonction du volume du liquide dans la carpule 11. Elle dépassera de ladite carpule d'une longueur permettant la translation du piston 23 sur une distance au moins suffisante au déplacement de la quantité de liquide nécessaire à la purge.

Ce premier conteneur **11** porte, de plus, fixé à l'extrémité distale en verre de la carpule, un appuie-doigt 26. Cet appuie-doigt est la zone où vient appuyer le pouce lors des opérations d'activation ou de reconstitution extemporanée. Il pourra avoir les différentes fonctions suivantes : il termine la carpule **11** comme n'importe quelle seringue ; dans sa fonction de tige de piston du dispositif, il évite que, lors de la pression du pouce, ne se crée une cavitation par le déplacement du piston 23, par exemple à l'aide de petites ouvertures ou fuites situées sous la zone d'appui et communiquant avec l'intérieur de la carpule 11, en arrière du piston 23. Il offre une prise en main lors du déverrouillage du second conteneur 13, après reconstitution grâce à un mouvement vers l'arrière de l'ensemble **11.** Enfin, l'appuie-doigt **26** peut servir à verrouiller ou bloquer le piston 23 et sa tige de purge 25 vers l'arrière pour empêcher qu'ils ne puissent être extraits de la carpule 11 ou que la tige de purge 25 ne soit dévissée.

Le second élément du dispositif formant le corps de la seringue s'articule autour de la carpule **13** contenant la partie solide ou lyophilisée **14** de la formulation conditionnée sous vide. Cette carpule 13 peut également être fermée à ses deux extrémités de façon standard. L'extrémité proximale, en regard de l'aiguille d'injection, peut être bouchée par une membrane ou pastille. Selon un mode de réalisation avantageux permettant un traitement standard en lyophilisation, elle sera fermée par un bouchon 27 engagé dans le col de la carpule. Ce bouchon 27 pourra assurer une meilleure étanchéité de la fermeture et le maintien du vide dans la carpule 13 ; il permettra éventuellement de réduire les volumes morts de la carpule d'injection ; il pourra, enfin, être utilisé comme les bouchons classiques des flacons de formes lyophilisées, c'est-à-dire être prépositionné après remplissage du milieu libérant alors des fentes ou fuites pour l'élimination des liquides, par sublimation lors de la lyophilisation et pourra ensuite boucher la carpule sous vide dans le lyophilisateur selon un mécanisme habituel. Cette possibilité standard n'est, toutefois, pas celle qui est décrite dans le procédé de conditionnement selon l'invention.

Ce procédé de conditionnement selon l'invention constitue un nouveau mode de réalisation avantageux en ce qu'il permet de pré-sceller la carpule au niveau de son col, par exemple avec une membrane ou pastille standard, sertie par une bague métallique, puis de la remplir et de la lyophiliser par l'arrière avant de la boucher par son piston d'injection habituel. Ainsi, les éléments de fermeture sont tout à fait standard ; on opère avec facilité du côté de l'ouverture la plus grande et on évite tout volume mort.

La membrane ou le bouchon **27** sont sertis sur le col par une capsule **28** qui peut être métallique. Le biseau interne de l'aiguille d'injection **20** connectera la formulation à l'aiguille, à travers cette membrane ou ce bouchon, au moment de l'injection.

A son autre extrémité, le conteneur **13** est bouché par un piston **29** qui pourra être un piston standard de carpules en caoutchouc ou en polybromobutyle. Ce piston **29** comporte un pas de vis interne **30** ou tout autre type de fixation susceptible de le solidariser avec une partie du verrou **16** formant le mécanisme de verrouillage - déverrouillage avec les clés **15** et **17.**

Ici, par exemple, la base du verrou **16** est vissée sur le pas de vis du piston **29.** Ce verrou **16** de même que les clés supérieure et inférieure sont creux de façon à contenir, à laisser circuler et à contrôler le déplacement de l'aiguille de transfert **18** lors de l'activation par transfert du liquide. C'est la carpule **11** dans sa fonction de piston qui va activer l'ensemble de ce mécanisme de transfert par la partie de son col qui s'y trouvera connectée. Le verrou **16** comporte, à son autre extrémité, des bras d'appui qui viendront reposer sur la base en verre de l'extrémité distale de la carpule **13** en position de verrouillage. Ces bases empêchent que la pression générée par le vide dans la carpule n'entraînent le piston vers le fond.

Autour des bras d'appui du verrou s'articule la clé inférieure **17.** Cette clé est libre en position verrouillée. Lors de l'activation, par enfoncement de la clé supérieure **15,** cette clé inférieure se retrouve solidaire à la partie proximale de la clé supérieure. A ce moment, les bras d'appui du verrou 16 bloquent le mouvement d'enfoncement, ne rendant plus possible qu'un mouvement de retrait de l'ensemble carpule **11** plus éléments de transfert vers l'arrière.

Lors de ce mouvement de retrait, la clé inférieure **17** libérera le verrou **16** par serrage des bras du verrou et blocage du mécanisme de transfert permettant ensuite l'injection.

L'aiguille de transfert **18** à l'intérieur du mécanisme de transfert et plus précisément de la clé supérieure **15,** comporte une embase **31** dont le rôle est de venir clipper l'aiguille 18 dans la clé 15 seulement après remplissage de ladite aiguille par le liquide 12 et avant percement du piston 29.

Pour un fonctionnement totalement fiable de l'activation, la longueur, le diamètre et le type de biseau, côté carpule et côté piston, pourront être optimisés de telle manière que l'aiguille de transfert pénètre systématiquement, d'abord à travers la membrane 21 de la carpule 11 et, seulement ensuite, à travers le piston 29. Par exemple, l'aiguille pourra être plus fine du côté carpule **11** avec un biseau long, plus pénétrant.

De même et selon les mêmes objectifs, ainsi que pour garantir plus d'étanchéité lors de la pénétration dans la chambre sous vide, la membrane 21 pourra être plus fine que le fond du piston 29. Ce dernier présentera une épaisseur suffisante à garantir l'étanchéité lors de la pénétration de l'aiguille 18 et, notamment, une épaisseur supérieure à la longueur du biseau de cette aiguille, côté piston 29.

Le mécanisme de déverrouillage vers l'arrière permet également, après réhydratation VAC, de retirer l'aiguille 18 du piston 29. Lors de l'injection, l'aiguille 18 restera bloquée dans la clé supérieure **15** et le blocage du mécanisme par le retrait ou serrage des bras du verrou 16, empêchera ladite aiguille 18 de retraverser le piston 29.

Cet ensemble carpule plus éléments de transfert est contenu dans le boîtier ou fourreau 19 qui forme le corps de la seringue. Selon un mode préférentiel d'arrangement dudit fourreau, il est constitué de trois sous-ensembles verrouillés ou clipés les uns dans les autres au moment du montage.

La partie principale ou centrale du fourreau **19** contient la carpule **13.** A son extrémité distale est fixée l'extrémité du fourreau 32 qui pourra comporter la partie appuie-doigt de la seringue ou poignée 33. Cette extrémité pourra, de plus, présenter le mécanisme de verrouillage et de blocage vers l'arrière de la carpule 13 et du mécanisme de transfert, empêchant, ainsi, tout retrait après montage.

A l'extrémité proximale de la partie centrale 19, se fixe l'extrémité ou bouchon du fourreau 34. C'est sur cette extrémité que vient se fixer, dans la version de la figure 1, l'aiguille d'injection 20 au niveau de son embase 35. C'est également au niveau de l'extrémité 34 que pourra se fixer le bouchon ou capuchon protecteur de l'aiguille 36.

Cette solution permet de réaliser un dispositif tout équipé et notamment à aiguille d'injection pré-montée sans avoir de problème avec la longueur et la position de ladite aiguille lors du conditionnement de la formulation.

Dans la version présentée, ce capuchon est bloqué par un verrou **37** que retient la tête de carpule **13.** De cette manière, le capuchon **36** ne pourra pas être retiré ni la carpule **13** être connectée à l'aiguille d'injection **20** avant que le mécanisme de transfert ait été entièrement réalisé c'est-à-dire jusqu'au retrait, après hydratation VAC.

Les autres fonctions spécifiques de certains éléments de l'ensemble seront précisées dans la description des figures qui y correspondent.

Une variante essentielle de la figure 1, en cas d'utilisation pour un système multidose du type stylo-injecteur, pourrait être la fixation d'aiguilles interchangeables au niveau de l'extrémité bouchon du fourreau **34** qui fermerait alors l'extrémité du stylo-injecteur.

Dans cette version, lors du retrait après réactivation, la carpule **11** pourrait être retirée et remplacée par un mécanisme classique de dosage et d'injection qui viendrait se fixer, par son conteneur, sur le fourreau **19** ou à son extrémité **32,** par exemple, par vissage. Ce conteneur du mécanisme de dosage et d'injection viendrait ainsi fermer le stylo-injecteur dans sa partie distale.

De la même manière que le vide dans les carpules assure un contrôle d'intégrité, on pourra emballer sous vide l'ensemble de la partie du corps de seringue du dispositif et obtenir, ainsi, le même contrôle, avec comme avantage un isolement thermique et une meilleure stabilité ou inertie. Ce vide de l'emballage pourra, par exemple, être supérieur au vide dans la carpule **13** pour assurer le relais, ou une double sécurité, sur la durée du stockage.

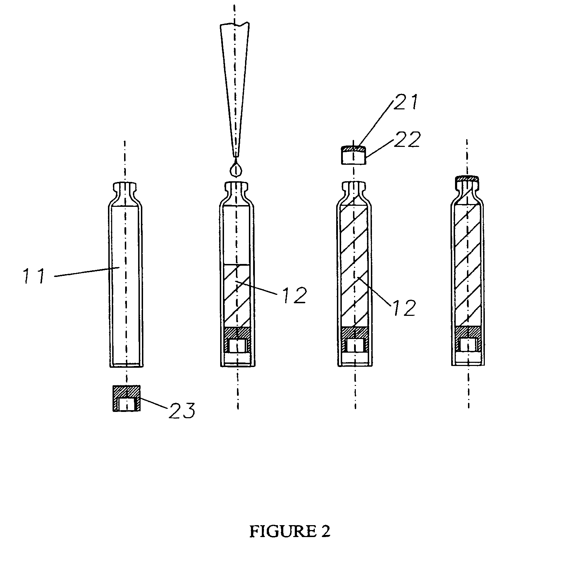

La figure 2 schématise un procédé classique de remplissage de la carpule **11** contenant la partie liquide de la formulation. Le piston **23** est préalablement installé et son positionnement dans le tube de la carpule **11** détermine exactement le volume liquide de reconstitution. Ce volume liquide **12** est ajouté, selon un procédé classique qui peut être réalisé sur des machines automatiques. La carpule **11,** une fois remplie à son bord jusque dans le col, est ensuite scellée par une carpule **22** contenant par exemple une membrane **21,** selon un mode également classique et automatisable. Ce procédé, qui pourra être réalisé par débordement, évite la présence de gaz à l'intérieur de la carpule scellée.

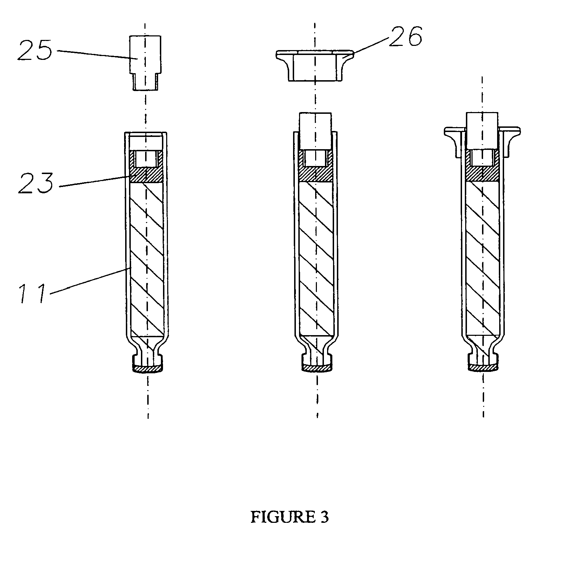

La figure 3 représente l'installation sur la carpule **11** de la tige de purge **25** par exemple par vissage sur le piston **23.** L'appuie-doigt **26** est ensuite fixé ou clipé sur la base en verre du tube de la carpule. Il pourra, ainsi, empêcher tout retrait du piston **23** et de la tige **25** et éviter le démontage de ladite tige **25.** L'ensemble ainsi assemblé pourra être classiquement stérilisé par exemple par autoclavage.

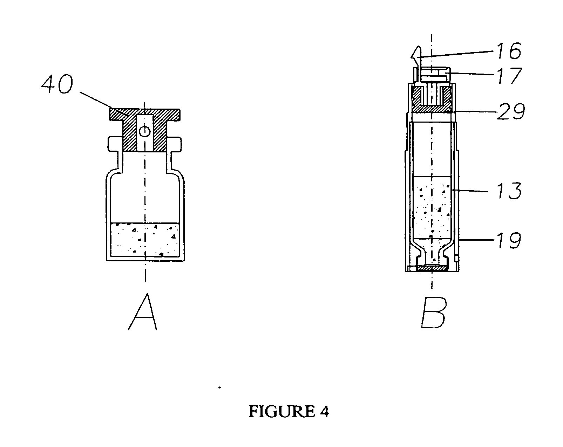

La figure 4 schématise une comparaison entre un flacon classique de lyophilisation et le dispositif, selon l'invention, dans ce qui constitue une partie de l'invention correspondant au procédé de lyophilisation et de conditionnement par l'arrière ou par la partie distale opposée au côté proximal de l'injection et du col de carpule. Dans un flacon classique **A,** le bouchon **40** est percé de trous et de fentes permettant son positionnement sur le col avant lyophilisation et sa fermeture par pression des étagères dans le lyophilisateur. L'objectif du dispositif, selon l'invention, est de permettre une telle lyophilisation non plus avec un flacon classique du type **A** mais avec une carpule standard **13** comme représentée en **B.** Pour ce faire, comme nous l'avons expliqué précédemment, il est possible d'utiliser un bouchon sur le col de la carpule **13** équivalent, en plus petit, au bouchon **40** du flacon **A.** Dans ce cas, le mécanisme de transfert où au moins un élément de verrou fixé au piston **29** est pré-positionné à l'extrémité distale du tube de la carpule **13** et la lyophilisation est faite par l'ouverture du col. Cette solution éloigne le lyophilisat de l'étagère d'échange calorique et l'isole partiellement par le piston **29** rendant, ainsi, plus difficile le procédé. De plus, elle complique le remplissage par le col étroit et elle ne permet pas un remplissage à 100 %.

Dans la solution du procédé de l'invention représentée ici, notamment pour optimiser et pour faciliter le remplissage de la carpule **13** et pour faciliter toutes opérations sur la préparation du lyophilisat, on opère de façon différente. La carpule est préalablement scellée avec son bouchon **27** par une capsule **28** (cf. figure 1) ; elle est remplie de la préparation à lyophiliser par sa grande ouverture distale et ainsi jusqu'au fond du col. Il est ainsi possible d'utiliser toutes les carpules standard pour y conditionner une forme lyophilisée, même les plus petites, et également d'opérer tous les arrangements possibles du futur lyophilisat, tels que par exemple, le remplissage de deux couches successives soit parce qu'elles sont incompatibles, soit parce que la seconde sert à éviter les volumes morts d'injection. Le piston d'injection **29** viendra boucher la capsule en fin de lyophilisation ; contrairement au bouchon **40,** il n'est pas équipé de fuites ou d'ouvertures synonymes de volume mort et il n'est pas prépositionné sur la base en verre de la carpule **13** mais sur un support de carpule **19** dans cette position inversée, qui lui sert de guidage. Ce support de carpule pourra avantageusement être la partie centrale du fourreau **19** ou corps de la seringue. L'avantage est d'avoir, ici, une forme parfaitement cylindrique qui économisera au maximum l'espace dans le lyophilisateur de même que le remplissage total du volume utile de la carpule **13.** Le fourreau **19** dans sa partie distale présente des zones de clips avec la partie **32** du corps de la seringue qui vont servir, ici, à la fois de maintien au piston **29** et d'ouverture à la carpule pendant la lyophilisation. Le piston **29** est pré-monté dans cette partie distale du fourreau **19** au moins avec une partie du mécanisme de transfert, à savoir, ici, le verrou **16** et la clé inférieure 17. Ce guidage du fourreau 19, une fois le dispositif assemblé, servira, ici, de blocage ou de butée au mouvement de la carpule 13 dans le corps de la seringue.

Cette lyophilisation à l'envers présente, donc, toute une somme d'avantages tout en permettant un procédé standard avec un bon contact avec la source froide.

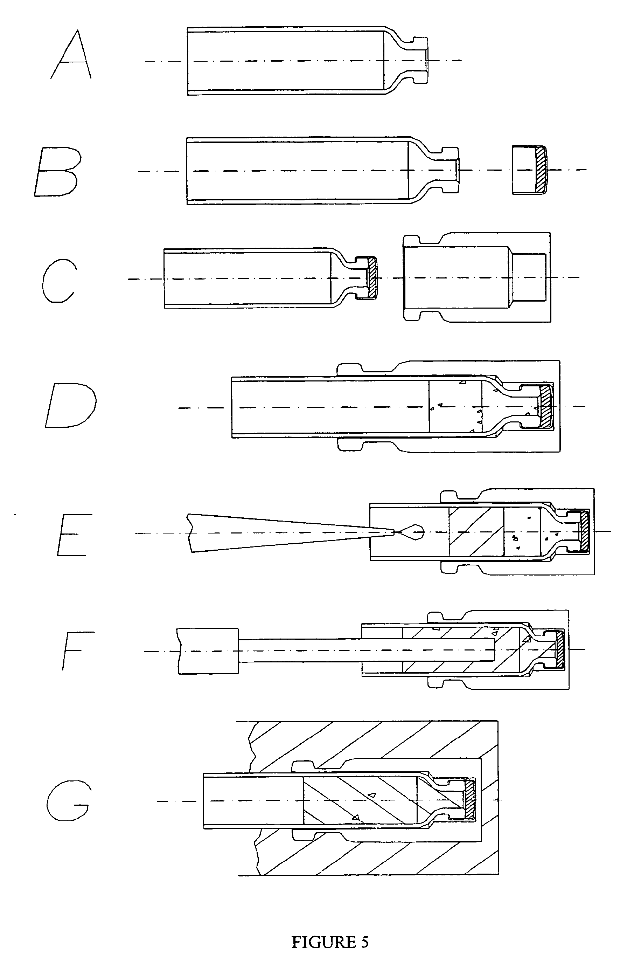

La figure 5 illustre la réalisation d'une opération spécifique de conditionnement d'une formulation à lyophiliser réalisée dans une carpule selon un procédé équivalent à celui traditionnellement utilisé avec un flacon. En A, la carpule 13 est lavée et siliconisée, en B, elle est sertie par sa capsule et membrane, en C, elle peut être installée sur un leurre reprenant la forme du flacon 40, en D et E, les constituants du mélange sont facilement introduits par la base, en F et G, on pourra réaliser tous traitements équivalents au flacon avec la même garantie de hauteur jusqu'à l'extérieur. Ici, est schématisé un traitement aux ultrasons. De la même manière, on pourrait agiter vigoureusement le mélange (vortex) ou la suspension avant congélation.

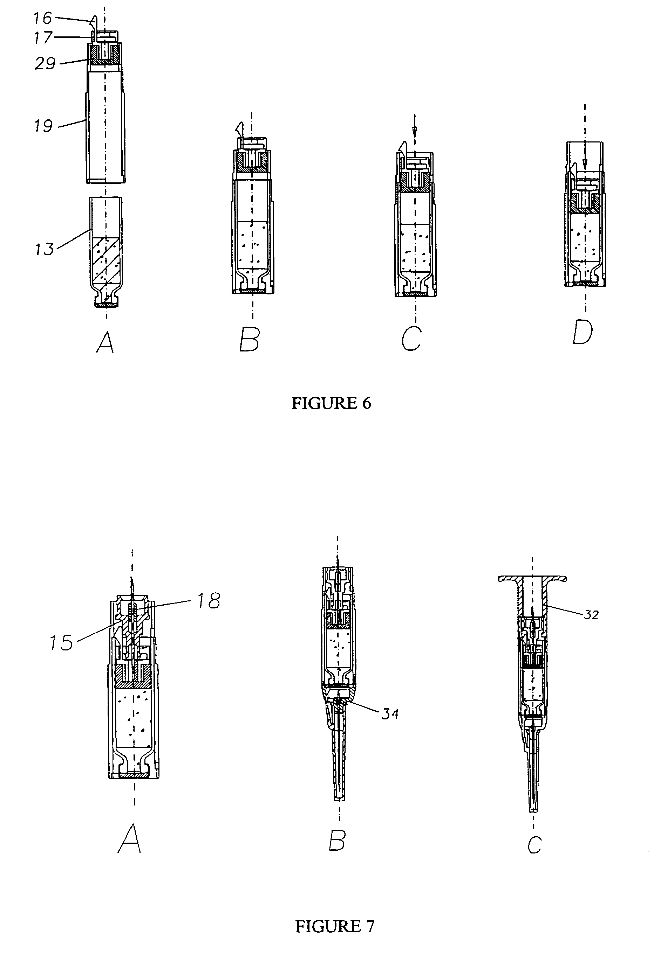

La figure 6 représente une carpule 13 remplie du mélange à lyophiliser préalablement congelée ou non. En A, cette carpule 13 est introduite dans le fourreau 19 qui lui sert de support. En B, l'ensemble des étapes du cycle de lyophilisation est normalement réalisé dans le lyophilisateur. En C, en fin de lyophilisation, et toujours sous vide, le piston 29 est introduit à l'intérieur du tube de la carpule 13 par pression classique dans le lyophilisateur sur le verrou 16. A ce moment, le lyophilisat est totalement isolé de l'extérieur par les lèvres du piston 29 en contact étanche avec la paroi du tube de la carpule. En D, on va rompre le vide à l'intérieur du lyophilisateur assurant, ainsi un retour, à la pression atmosphérique. Sous l'action de cette pression, le piston 29 et le verrou 16 vont continuer la descente dans le tube de la capsule 19 assurant, de ce fait, un contrôle visuel et individuel de l'existence et de la qualité du vide dans la carpule. Ce positionnement du piston va venir s'y arrêter par l'appui du bras de verrou 16 sur la carpule 13. L'élément scellé, ainsi constitué, fait ensuite partie intégrante du dispositif selon l'invention.

La figure 7 représente la suite des opérations d'assemblage dudit dispositif. En A, sur la base de l'élément D de la figure 6, sont assemblées les autres parties du mécanisme de transfert. A savoir, la clé supérieure 15 dans laquelle est pré-montée l'aiguille de transfert 18. Cette clé supérieure pourra avoir une couronne de clip pour la tête de la carpule 11, plus haute autour de l'aiguille pour la protéger lors de ses manipulations. En B, on fixe, par exemple, par clipage la partie proximale ou bouchon du fourreau 34 qui inclut préalablement l'aiguille d'injection 20 et le capuchon 36. En C, on fixe, enfin, la troisième partie du corps de la seringue à savoir la partie distale du fourreau 32. Cette partie du corps de la seringue pourra, ensuite, être emballée individuellement et être post-stérilisée, par exemple par gamma-irradiation.

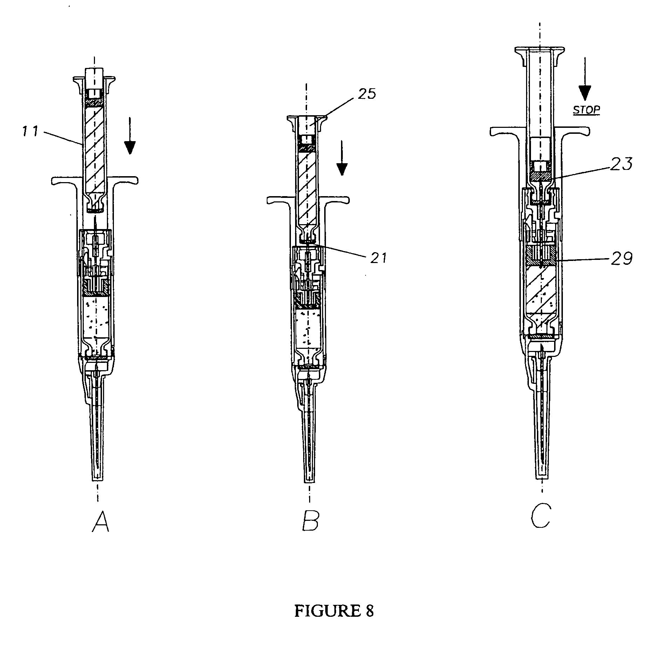

La figure 8 représente les étapes de reconstitution de la formulation contenue dans le dispositif de l'invention. En **A,** on introduit la carpule **11** dans le corps de la seringue. En B, on active la reconstitution par pression sur la tige de purge 25. La première opération est alors le percement du disque 21 puis le remplissage de l'aiguille. En C et selon le même geste de pression sur la tige, on opère, enfin, le percement du piston 29. A ce moment, le geste est alors bloqué et la préparation se reconstitue automatiquement avec descente du piston 23 dans le tube de la carpule 11.

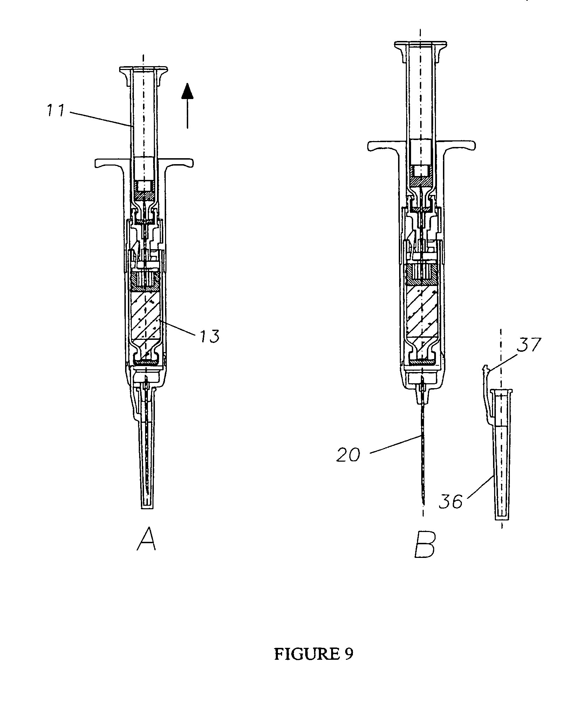

La figure 9 représente tout d'abord, à partir de l'étape C de la figure 8, en A, le déblocage par retrait de la tige de piston constituée par la carpule **11.** Cette opération a préalablement été décrite dans le fonctionnement du mécanisme de transfert. Elle entraîne un peu la carpule 13 vers l'arrière jusqu'à sa butée sur le fourreau. En B, on opère alors le décapuchonnage rendu possible par la libération du capuchon 36 au niveau de son blocage 37, opéré par le léger retrait de la tête de la carpule d'injection 13. A ce moment seulement, la carpule d'injection 13, peut être connectée à l'aiguille d'injection 20.

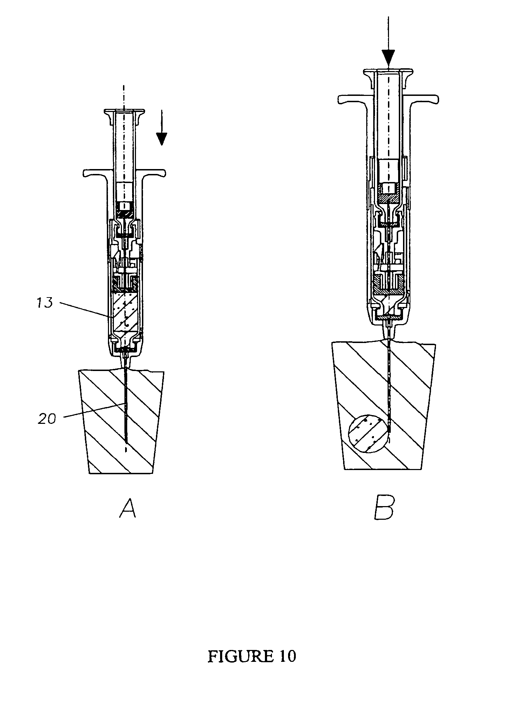

La figure 10 schématise l'injection selon un mode tout à fait standard. Étant donné que le dispositif, selon l'invention, permet une préparation qui ne nécessite pas de purge préalable à l'injection, il est possible de pratiquer, avant A, l'injection de l'aiguille 20 préalablement à sa connexion à la carpule 13. Cette étape peut également servir de test de veine direct, par exemple, en cas d'injection IV ou IM par visualisation d'un retour éventuel de sang du côté du biseau interne de l'aiguille avant connexion à la formulation. En B, en fin d'injection, le mécanisme pourra être bloqué pour éviter toutes manipulations postérieures.

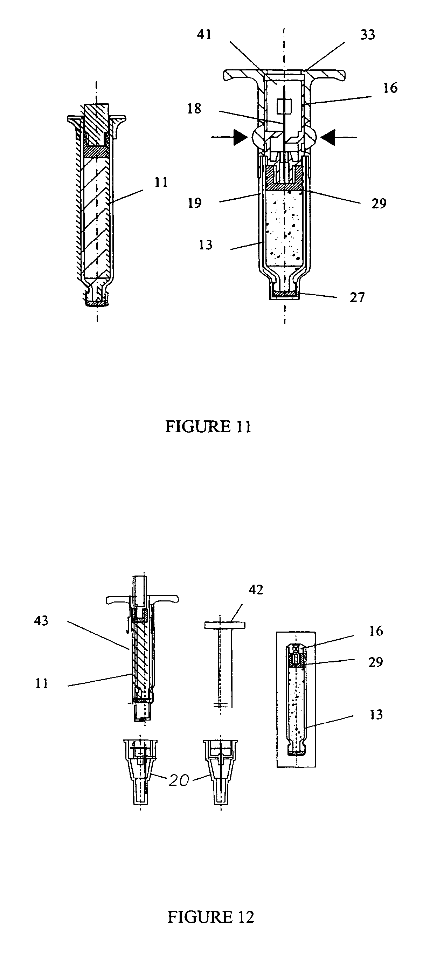

La figure 11 représente une autre réalisation possible du dispositif, selon l'invention, adaptable aux traitements, en condition aseptique (notamment au remplissage) et aux volumes plus faibles. La mise en oeuvre de ce dispositif ne nécessite aucun montage aseptique après fermeture dans le lyophilisateur, le dispositif et ses éléments constitutifs étant alors isolés de l'extérieur.

Lors du procédé de lyophilisation, selon l'invention, la carpule de lyophilisat 13 est installée à l'intérieur du fourreau 19 qui est, alors, positionné en position proximale autour du col de la carpule dont il est le support et qui porte à son extrémité distale, le verrou 16 fermé à une extrémité par le piston 29 et à l'autre extrémité par un film 15 avec à l'intérieur l'aiguille de transfert 18.

En fin de lyophilisation, le réservoir est fermé par le piston **29** en appuyant sur le verrou 16 et grâce aux zones de guidage du fourreau 19 (non représentées). Le dispositif est alors définitivement verrouillé par la tête de fourreau 27 et l'appuie-doigt 33.

Le traitement de la carpule de liquide 11 est similaire à l'exemple détaillé précédemment. Au moment de l'utilisation, la carpule 11 est introduite à travers le film 41. Elle se connecte et purge l'aiguille 18 avant de percer le piston 29 pour la réhydratation VAC.

Une fois cette opération réalisée, le verrou 16 est déverrouillé en appuyant sur la zone des flèches du fourreau 19, entraînant ainsi la rupture de parois fines et le clipage de 2 bras qui retiendront le verrou et courberont l'aiguille 18 permettant alors son extraction du piston 29. L'aiguille d'injection peut alors être installée sur la tête 27 et l'injection être pratiquée par la carpule 11 servant de tige au piston 29.

La figure 12 illustre une autre représentation du dispositif, selon l'invention du procédé de conditionnement par l'arrière d'une forme solide et de son procédé de réhydratation à travers le piston d'injection du dispositif.

Ici, le procédé de lyophilisation par l'arrière, selon l'invention, est pratiqué sur les carpules 13 préalablement operculées et installées dans un support inférieur (non représenté) indexé au support supérieur (non représenté) des piston 29 et verrou 16. En fin de lyophilisation, le piston 29 et son verrou 16 sont introduits dans la carpule 13. Une autre solution préférable, selon le procédé de l'invention consiste à remplacer ces supports inférieurs et supérieurs indexés par des supports individuels (21) pour chaque carpule. Ces supports individuels peuvent alors remplir la fonction du leurre telle que décrite dans la figure 5. De plus, ces supports peuvent être directement utilisés dans le lyophilisateur et peuvent constituer la base d'un emballage réalisé avec le support individuel supérieur (22) du piston 29 et du verrou 16.

Après lyophilisation, les supports individuels inférieur (21) et supérieur (22) se referment pour constituer dans le lyophilisateur, l'emballage de la carpule.

La carpule de liquide 11 est préparée comme précédemment décrit, elle peut présenter une tige de purge mais pas nécessairement d'appuie-doigt. Un fourreau 43 et une tige de piston 42 peuvent également faire partie du dispositif. Deux aiguilles d'injection 20 (représentées ici dans leur capuchon) du type aiguilles jetables pour stylo-injecteur (par exemple Micro Fine® de Becton Dickinson) sont également nécessaires à son utilisation.

Dans le cas de l'utilisation de ce dispositif comme seringue unidose, la carpule **11** est introduite dans le fourreau **19** comme représenté sur la figure **12,** une aiguille **20** est fixée et la réhydratation est pratiquée en perçant le piston **29** à travers le verrou **16.**

Le fourreau **19** est ensuite séparé de la carpule **11** et le verrou **16** est dévissé grâce à l'embase de l'aiguille **20** puis jeté dans le capuchon. Le fourreau **19** peut présenter des ouvertures latérales permettant le dégagement de la carpule **11** et sa réutilisation pour l'injection de la préparation reconstituée dans la carpule **13** grâce à l'adjonction d'une tige de piston **42** sur le piston **29** et à la seconde aiguille **20.**

Dans le cas de l'utilisation de ce dispositif comme carpule de stylo-injecteur multidose, la carpule **11** est introduite dans la partie proximale du stylo que pourrait symboliser ici le fourreau **19,** une aiguille **20** y est fixée et la réhydratation et le retrait du verrou **16** sur la carpule **13** sont pratiqués comme précédemment. La carpule **13** remplace alors la carpule **11** dans la partie proximale du stylo pour une utilisation classique de celui-ci comme avec les carpules de produits en solution.

La figure **13** présente une application simplifiée du dispositif selon l'invention dans le cas d'une formulation équivalente (en volume et composition) à celle de l'exemple détaillé. Le dispositif peut donc fonctionner avec les mêmes carpules **11** pour la partie liquide de la formulation **12** et **13** pour la partie lyophilisée **14** que celles représentées sur la figure 1.

La différence essentielle se trouve au niveau du verrou connecteur **16** qui est vissé au piston d'injection **29** et constitué d'une seule pièce. Aucune opération de montage à ce niveau n'est nécessaire avant stérilisation. De même, il est possible d'éviter l'emballage avant gamma-irradiation.

La carpule **13** est installée avant lyophilisation dans le fourreau **19** qui servira de guide lors de la fermeture du piston **29.** La tête de seringue **34** constitue l'embase de l'aiguille **20.** Elle peut être montée étanche avec le capuchon **36** sur le fourreau **19** par exemple grâce à un joint torique entre la carpule **13** et le fourreau **19.**

Dans ce cas, aucun emballage n'est nécessaire et le dispositif est clos entre le capuchon **36** et le verrou connecteur **16.** L'appuie-doigt **33** peut être clippé sur la partie distale du fourreau **19.**

L'utilisation de ce dispositif, selon l'invention, consiste à fixer une aiguille standard **18** du type aiguilles jetables pour stylo-injecteur (par exemple Micro Fine® de Becton Dickinson) sur le pas de vis du fourreau contenant la carpule de liquide **11** ou, comme représenté ici, sur le seul pas de vis **44** fixé de façon solidaire à la carpule **11.** L'ensemble est alors introduit dans le verrou connecteur 16 puis à travers le piston **29** en appuyant sur la tige de purge **25.** Après réhydratation VAC, la carpule **11** est retirée par dévissage avec l'aiguille de transfert **18** et le verrou **16 ;** une autre solution consiste à rallonger les bras de maintien du verrou sur la carpule de façon à clipper les bras du verrou connecteur **16** à son niveau, seulement après disparition de l'aiguille dans ledit verrou. L'ensemble carpule-verrou connecteur est alors dévissé du piston 29. Une tige de piston, qui pourra être le capuchon **36,** est fixée au piston pour pratiquer l'injection.

### Principaux modes de réalisation préférés de l'invention

L'invention concerne donc tout d'abord un dispositif du type seringue tel que défini dans la revendication 1.

Selon des variantes préférées de l'invention, ledit dispositif peut présenter additionnellement au moins une des caractéristiques complémentaires suivantes :
1) ledit liquide et ledit non liquide sont conditionnés à l'intérieur de réservoirs classiques d'injection ;
2) ledit dispositif possède la caractéristique 1) et est en outre une seringue unidose ou multidose du type stylo-injecteur ;
3) le transfert lors de ladite reconstitution est effectué à travers une pointe, un canal, un tube ou une aiguille de transfert creuse introduit dans ledit piston ;
4) ledit piston présente un mécanisme de blocage sur ledit réservoir empêchant son enfoncement avant et pendant ladite reconstitution de ladite préparation, ledit mécanisme de blocage pouvant optionnellement également être le connecteur de ladite partie liquide sur ledit réservoir de ladite seringue ;
5) ledit dispositif possède la caractéristique 4) décrite ci-dessus et ledit mécanisme de blocage est vissé sur ledit piston ;
6) ledit dispositif possède les caractéristiques 4) ou 5) et ledit mécanisme de blocage peut être désactivé, de préférence après la reconstitution de ladite préparation ;
7) ledit dispositif possède la caractéristique 6) et la désactivation dudit mécanisme de blocage correspond également au retrait dudit élément de transfert dudit piston ;
8) ladite partie liquide est dans un conteneur servant à activer ledit transfert dans ledit réservoir de ladite seringue à travers ledit mécanisme de blocage ;
9) ledit dispositif possède la caractéristique 8), ledit conteneur servant ensuite en outre de tige d'injection audit piston ;
10) ledit dispositif possède la caractéristique 9), lesdits réservoirs classiques étant des carpules en verre ;
11) ledit liquide est conditionné dans un réservoir dont le piston est activé par une courte tige permettant d'initier le déplacement dudit piston et de purger ledit élément de transfert avant injection dans ledit réservoir dudit non liquide ;
12) ledit dispositif possède la caractéristique 11), ledit réservoir dudit liquide étant en outre équipé d'une extrémité évitant son obturation par le pouce de la main ;
13) ladite partie non liquide dans ledit réservoir est conditionnée sous vide ;
14) ledit dispositif possède la caractéristique 13), ladite partie liquide dans ledit second réservoir contrôlant en outre automatiquement, par son volume, le volume reconstitué de ladite préparation ;
15) ledit dispositif possède la caractéristique 13) ou la caractéristique 14), la reconstitution de ladite préparation étant opérée automatiquement en appuyant sur ledit second réservoir ;
16) ledit dispositif, possédant éventuellement une ou des caractéristiques choisies parmi les caractéristiques 1) à 15), constitue une seringue unidose d'injection ;
17) ledit dispositif, possédant éventuellement une ou des caractéristiques choisies parmi les caractéristiques 1) à 14), constitue une seringue multidose du type stylo-injecteur ;
18) ledit dispositif possède la caractéristique 17), ledit second réservoir étant retiré après ladite reconstitution et remplacé par le mécanisme de dosage ;
19) au moins ledit réservoir dudit dispositif contenant ladite partie non liquide est ensuite emballé sous vide ;
20) l'aiguille d'injection est préfixée et son capuchon bloqué par un mécanisme de blocage avant la reconstitution de ladite préparation.

Selon une variante particulière de l'invention, le piston présente un élément de transfert creux et un mécanisme de blocage sur le réservoir. De préférence, ledit mécanisme de blocage est creux et contient ledit élément de transfert.

L'élément de transfert peut être, par exemple, une aiguille, une pointe, un canal ou un tube. De préférence, il s'agira d'une aiguille.

L'invention concerne également un procédé de conditionnement selon le revendication 13.

Selon des variantes préférées de l'invention, ledit procédé peut présenter additionnellement au moins une des caractéristiques complémentaires suivantes :
a) ledit réservoir cylindrique est préalablement introduit dans un support sur lequel ledit piston est préalablement positionné, permettant ainsi une ouverture vers l'extérieur dudit réservoir à travers ledit support, ledit piston étant ensuite introduit dans ledit réservoir par pression et par déplacement dans ledit support qui lui sert de guidage ;
b. ledit procédé possède la caractéristique a) ledit piston étant ensuite définitivement positionné dans ledit réservoir par la pression ambiante permettant ainsi un contrôle de conditionnement ;
c) ledit procédé possède la caractéristique a) ou b), ledit support faisant ensuite partie intégrante du dispositif final ;
d) lesdits éléments à conditionner sont un mélange préalablement agité de façon très vigoureuse dans ledit réservoir ;
e) ledit procédé possède l'une des caractéristiques a) à e), lesdits éléments étant introduits et congelés séparément en couche dans ledit réservoir avant ladite lyophilisation ;
f) ledit réservoir cylindrique est préalablement introduit dans un support et ledit piston bloqué dans un autre support, permettant ainsi une ouverture vers l'extérieur dudit réservoir entre les deux supports, ledit piston étant ensuite introduit dans ledit réservoir par pression et par rapprochement desdits deux supports ;
g) ledit procédé possède la caractéristique f), lesdits supports étant en outre hermétiquement clos après ledit rapprochement et constituant l'emballage dudit réservoir cylindrique.

Toutes les variantes exposées précédemment ne représentent qu'une partie des réalisations mises à la disposition de l'homme du métier à la lumière de la présente description et ne sauraient limiter la portée de la présente invention. Le spécialiste saura bien sûr adapter à ses besoins spécifiques l'enseignement qui lui est donné ici.

## Revendications

1. Dispositif du type seringue actionné par un piston pour reconstituer une préparation, en solution, en suspension ou en dispersion dans un liquide à l'intérieur d'un réservoir proximal (13) fermé à une extrémité par un élément fixe (27) et à l'autre extrémité par un piston (29) et formant partie du corps de ladite seringue, ledit dispositif comportant:
- une carpule constituant le réservoir proximal (13) et contenant une partie non liquide (14), ici le lyophilisat de ladite préparation qui a été préalablement conditionnée sous vide à l'intérieur dudit réservoir proximal (13) de ladite seringue; une carpule constituant un réservoir distal (11) contenant une partie liquide (12) de ladite préparation, préalablement séparée dudit réservoir proximal et de ladite seringue;
- ledit piston (29) qui comporte un pas de vis interne (30) ou tout autre type de fixation susceptible de le solidariser avec la partie d'un verrou (16) formant un mécanisme de blocage sur ledit réservoir proximal (13);
- ledit verrou (16), qui comporte a une extrémité des bras d'appui pouvant reposer sur une base d'une extrémité distale de ladite carpule constituent le réservoir proximal (13) lors de la position de verrouillage empêchant que le vide dans la carpule (13) n'entraîne le piston vers le fond avant et pendant ladite reconstitution, de façon à ce que
la reconstitution de ladite préparation est effectuée par transfert de ladite partie liquide (12) à travers ledit piston (29) dudit réservoir (13) à l'aide d'un élément de transfert creux (18) introduit dans ledit piston (29).

2. Dispositif selon la revendication 1, **caractérisé en ce que** ledit verrou formant le mécanisme de blocage (16) est creux et contient ledit élément de transfert (18).

3. Dispositif selon la revendication 1 ou 2, **caractérisé en ce que** ledit mécanisme de blocage (16) peut être désactivé lors du mouvement de retrait de l'ensemble de la carpule constituant le réservoir distal (11) vers l'arrière.

4. Dispositif selon la revendication 3, **caractérisé en ce que** la désactivation dudit verrou formant le mécanisme de blocage (16) est produit par le retrait de la dite carpule (11) vers l'arrière libérant ledit verrou (16) par l'intermédiaire d'une clé inférieure (17).

5. Dispositif selon la revendication 4, **caractérisé en ce que** la libération dudit verrou (16) bloque le mécanisme de transfert (18) permettant l'injection.

6. Dispositif selon l'une des revendications 1 à 5 **caractérisé en ce que** l'élément de transfert est une aiguille (18).

7. Dispositif selon l'une quelconque des revendications précédentes, **caractérisé en ce que** ladite partie liquide (12) est contenue dans une carpule formant un réservoir distal (11) servant à activer ledit transfert dans ledit réservoir proximal (13) de la seringue à travers ledit mécanisme de blocage (16).

8. Dispositif selon l'une des revendications 1 à 7, **caractérisé en ce que** :
- ladite partie liquide (12) et ladite partie non liquide (14) sont conditionnés à l'intérieur de réservoirs classiques d'injection (11, 13) ; et
- ledit dispositif est une seringue unidose ou multidose du type stylo-injecteur.

9. Dispositif selon l'une quelconque des revendications précédentes, **caractérisé en ce que** ladite partie non liquide (14) contenue dans ladite carpule formant le réservoir proximal (13) est conditionnée sous vide.

10. Dispositif selon la revendication 9, **caractérisé en ce que** ladite partie liquide (12) dans ladite carpule formant le réservoir distal (11) contrôle en outre automatiquement, par son volume, le volume reconstitué de ladite préparation.

11. Dispositif selon la revendication 9 ou 10, **caractérisé en ce que** la reconstitution de la préparation est opérée automatiquement en appuyant sur ladite carpule formant le réservoir distal (11).

12. Dispositif selon l'une des revendications précédentes, **caractérisée en ce que** son aiguille d'injection (20) est préfixée et son capuchon (36) bloqué par un mécanisme de blocage (37) avant la reconstitution de ladite préparation.

13. Procédé de conditionnement sous vide à l'intérieur d'une carpule formant un réservoir proximal (13) fermé d'un côté par un piston d'injection (29) bloqué dans son déplacement dans ledit réservoir proximal (13), dans lequel les parties non liquide (14) à conditionner sont introduits à l'intérieur dudit réservoir proximal (13) du côté dudit piston d'injection (29) et on procède à une byophilisation avant que ledit réservoir proximal (13) ne soit fermé par ledit piston (23), ledit réservoir (13) étant préalablement fermé de l'autre côté.

14. Procédé selon la revendication 13, **caractérisé en ce que** le piston (29) est ensuite définitivement positionné dans le réservoir proximal (13) par la pression ambiante, permettant ainsi un contrôle de conditionnement.

15. Procédé selon la revendication 13 ou 14, **caractérisé en ce que** les parties non liquide à conditionner (14) sont introduits et congelés séparément en couche dans le réservoir proximal (13) avant la lyophilisation.

16. Procédé selon l'une des revendications 13 à 15, **caractérisé en ce que** :
- ledit réservoir proximal (13) est préalablement introduit dans un support (19) sur lequel ledit piston (29) est préalablement positionné, permettant ainsi une ouverture vers l'extérieur dudit réservoir proximal (13) à travers ledit support (19) ; et
- ledit piston (29) est ensuite introduit dans ledit réservoir proximal (13) par pression et par déplacement dans ledit support (19) qui lui sert de guidage.

17. Procédé selon l'une des revendications 13 à 16, **caractérisé en ce que** :
- ledit réservoir proximal (13) est préalablement introduit dans un support (19) et ledit piston (29) bloqué, dans un autre support (19) permettant ainsi une ouverture vers l'extérieur dudit réservoir entre les deux supports (19) ; et
- ledit piston (29) est ensuite introduit dans ledit réservoir proximal (13) par pression et par rapprochement desdits deux supports (19).

18. Procédé selon l'une des revendications 16 ou 17, **caractérisé en ce que** lesdits supports (19) constituent ensuite une partie d'un dispositif seringue d'injection et de son emballage.

19. Procédé selon l'une des revendications 13 à 18, **caractérisé en ce que** les parties non liquide à conditionner (14) sont un mélange préalablement agité de façon très vigoureuse dans le réservoir.

20. Procédé selon l'une des revendications 13 à 19, **caractérisé en ce que** le réservoir cylindrique est préalablement introduit dans un support (19) et le piston (29) bloqué dans un autre support (19), permettant ainsi une ouverture vers l'extérieur dudit réservoir entre les deux supports (19), ledit piston étant ensuite introduit dans ledit réservoir par pression et par rapprochement desdits deux supports (19).

21. Procédé selon la revendication 20, **caractérisé en ce que** les supports (19) sont en outre hermétiquement clos après le rapprochement et constituent l'emballage du réservoir proximal (13).

## Claims

1. Device of syringe type actuated by a piston in order to reconstitute a preparation, in solution, in suspension or in dispersion in a liquid inside a proximal reservoir (13) sealed at one end by a fixed element (27) and at the other end by a piston (29) and forming part of the body of said syringe, said device comprising:
- a cartridge constituting the proximal reservoir (13) and containing a non-liquid part (14), in this case the lyophilisate of said preparation which has been packed beforehand under vacuum inside said proximal reservoir (13) of said syringe; a cartridge constituting a distal reservoir (11) containing a liquid part (12) of said preparation, separated beforehand from said proximal reservoir and said syringe;
- said piston (29) which comprises an internal screw-thread (30) or any other type of attachment capable of attaching it firmly to the part of a lock (16) forming a locking mechanism on said proximal reservoir (13);
- said lock (16), which comprises at one end of the support arms which can rest on a base of a distal end of said cartridge constituting the proximal reservoir (13) during locking position which prevents the vacuum in the cartridge (13) from carrying the piston downwards towards the bottom before and during said reconstitution,
- such that the reconstitution of said preparation is carried out by transfer of said liquid part (12) through said piston (29) of said reservoir (13) using a hollow transfer element (18) introduced into said piston (29).

2. Device according to claim 1, **characterized in that** said lock forming the locking mechanism (16) is hollow and contains said transfer element (18).

3. Device according to claim 1 or 2, **characterized in that** said locking mechanism (16) can be deactivated during a rearward movement withdrawing the assembly of the cartridge constituting the distal reservoir (11).

4. Device according to claim 3, **characterized in that** the deactivation of said lock forming the locking mechanism (16) is produced by the withdrawal of said cartridge (11) towards the rear releasing said lock (16) via a lower key (17).

5. Device according to claim 4, **characterized in that** the release of said lock (16) locks the transfer mechanism (18) allowing the injection.

6. Device according to one of claims 1 to 5, **characterized in that** the transfer element is a needle (18).

7. Device according to any one of the previous claims, **characterized in that** said liquid part (12) is contained in a cartridge forming a distal reservoir (11) serving to activate said transfer into said proximal reservoir (13) of the syringe through said locking mechanism (16).

8. Device according to one of claims 1 to 7, **characterized in that**:
- said liquid part (12) and said non-liquid part (14) are packed inside standard injection reservoirs (11, 13); and
- said device is a single-dose or multi-dose syringe of pen-injector type.

9. Device according any one of the previous claims, **characterised in that** said non-liquid part (14) contained in said cartridge forming the proximal reservoir (13) is packed under vacuum.

10. Device according to claim 9, **characterised in that** said liquid part (12) in said cartridge forming the distal reservoir (11) also automatically controls, by its volume, the reconstituted volume of said preparation.

11. Device according to claim 9 or 10, **characterised in that** the reconstitution of the preparation is carried out automatically by bearing on said cartridge forming the distal reservoir (11).

12. Device according to one of the previous claims, **characterised in that** its injection needle (20) is pre-fixed and its cap (36) locked by a locking mechanism (37) before the reconstitution of said preparation.

13. Vacuum packaging process inside a cartridge forming a proximal reservoir (13) sealed on one side by an injection piston (29) locked in its movement in said proximal reservoir (13), in which the non-liquid parts (14) to be packed are introduced into the inside of said proximal reservoir (13) from the side of said injection piston (29) and lyophilization is carried out before said proximal reservoir (13) is sealed by said piston (23), said reservoir (13) being sealed beforehand from the other side.

14. Process according to claim 13, **characterized in that** the piston (29) is then definitively positioned in the proximal reservoir (13) by the ambient pressure, thus allowing a packaging control.

15. Process according to claim 13 or 14, **characterized in that** the non-liquid parts to be packed (14) are introduced and frozen separately in layers in the proximal reservoir (13) before lyophilization.

16. Process according to one of claims 13 to 15, **characterized in that**:
- said proximal reservoir (13) is introduced beforehand into a support (19) on which said piston (29) is positioned beforehand, thus allowing an opening to the outside of said proximal reservoir (13) through said support (19); and
- said piston (29) is then introduced into said proximal reservoir (13) by pressure and by displacement in said support (19) which serves to guide it.

17. Process according to one of claims 13 to 16, **characterized in that**:
- said proximal reservoir (13) is introduced beforehand into a support (19) and said piston (29) locked in another support (19), thus allowing an opening to the outside of said reservoir between the two supports (19); and
- said piston (29) is then introduced into said proximal reservoir (13) by pressure and by a bringing together of said two supports (19).

18. Process according to one of claims 16 or 17, **characterized in that** said supports (19) then constitute a part of the injection syringe device and of its packaging.

19. Process according to one of claims 13 to 18, **characterized in that** the non-liquid parts to be packed (14) are a mixture stirred beforehand in a very vigorous manner in the reservoir.

20. Process according to one of claims 13 to 19, **characterized in that** the cylindrical reservoir is introduced beforehand into a support (19) and the piston (29) locked in another support (19), thus allowing an opening to the outside of said reservoir between the two supports (19), said piston then being introduced into said reservoir by pressure and by a bringing together of the two supports (19).

21. Process according to claim 20, **characterized in that** the supports (19) are moreover hermetically closed after bringing together and constitute the packaging of the proximal reservoir (13).

## Patentansprüche

1. Vorrichtung vom Typ Spritze, die durch einen Kolben betätigt wird, zur Rekonstituierung eines Präparats in Lösung, in Suspension oder in Dispersion in einer Flüssigkeit im Inneren eines proximalen Behälters (13), der an einem Ende durch ein feststehendes Element (27) und am anderen Ende durch einen Kolben (29) geschlossen ist und einen Teil des Körpers dieser Spritze bildet, wobei diese Vorrichtung umfasst:
- eine Karpule, die den proximalen Behälter (13) bildet und einen nicht flüssigen Teil (14), im vorliegenden Fall das Lyophilisat dieses Präparats, enthält, das zuvor unter Vakuum im Inneren dieses proximalen Behälters (13) der Spritze verpackt wurde;
- eine Karpule, die einen einen flüssigen Teil (12) des Präparats enthaltenden distalen Behälter (11) bildet und zuvor von dem proximalen Behälter und der Spritze getrennt ist;
- den Kolben (29), der einen inneren Schraubengang (30) oder jede andere Art von Befestigung umfasst, die ihn mit dem Teil eines Riegels (16) fest verbinden kann, der einen Mechanismus zur Blockierung an dem proximalen Behälter (13) bildet;
- den Riegel (16), der an einem Ende Stützarme umfasst, die auf einer Basis eines distalen Endes der den proximalen Behälter bildenden Karpule (13) in der Verriegelungsstellung aufliegen können, wobei sie verhindern, dass das Vakuum in der Karpule (13) den Kolben vor und während der Rekonstituierung zum Boden mitnimmt, so dass die Rekonstituierung des Präparats durch Übertragung dieses flüssigen Teils (12) durch den Kolben (29) des Behälters (13) hindurch mit Hilfe eines in den Kolben (29) eingeführten hohlen Übertragungselements (18) durchgeführt wird.

2. Vorrichtung nach Anspruch 1, **dadurch gekennzeichnet, dass** der den Blockiermechanismus (16) bildende Riegel hohl ist und das Übertragungselement (18) enthält.

3. Vorrichtung nach Anspruch 1 oder 2, **dadurch gekennzeichnet, dass** der Blockiermechanismus (16) bei der Auszugsbewegung der Einheit der den distalen Behälter (11) bildenden Karpule nach hinten deaktiviert werden kann.

4. Vorrichtung nach Anspruch 3, **dadurch gekennzeichnet, dass** die Deaktivierung des den Blockiermechanismus (16) bildenden Riegels durch die Rückbewegung der Karpule (11) nach hinten erzeugt wird, die den Riegel (16) über einen unteren Schlüssel (17) freigibt.

5. Vorrichtung nach Anspruch 4, **dadurch gekennzeichnet, dass** die Freigabe des Riegels (16) den die Injektion gestattenden Übertragungsmechanismus (18) blockiert.

6. Vorrichtung nach einem der Ansprüche 1 bis 5, **dadurch gekennzeichnet, dass** das Übertragungselement eine Nadel (18) ist.

7. Vorrichtung nach einem der vorhergehenden Ansprüche, **dadurch gekennzeichnet, dass** der flüssige Teil (12) in einer einen distalen Behälter (11) bildenden Karpule enthalten ist, die dazu dient, die Übertragung in den proximalen Behälter (13) der Spritze durch den Blokkiermechanismus (16) hindurch zu aktivieren.

8. Vorrichtung nach einem der Ansprüche 1 bis 7, **dadurch gekennzeichnet, dass**
- der flüssige Teil (12) und der nicht flüssige Teil (14) im Inneren von herkömmlichen Injektionsbehältern (11, 13) verpackt sind; und
- die Vorrichtung eine Einzeldosen- oder Mehrdosen-Spritze vom Typ Injektionsfüller ist.

9. Vorrichtung nach einem der vorhergehenden Ansprüche, **dadurch gekennzeichnet, dass** der nicht flüssige Teil (14), der in der den proximalen Behälter (13) bildenden Karpule enthalten ist, unter Vakuum verpackt ist.

10. Vorrichtung nach Anspruch 9, **dadurch gekennzeichnet, dass** der flüssige Teil (12) in der den distalen Behälter (11) bildenden Karpule außerdem automatisch durch sein Volumen das rekonstituierte Volumen des Präparats steuert.

11. Vorrichtung nach Anspruch 9 oder 10, **dadurch gekennzeichnet, dass** die Rekonstituierung des Präparats automatisch vorgenommen wird, indem auf die den distalen Behälter (11) bildende Karpule gedrückt wird.

12. Vorrichtung nach einem der vorhergehenden Ansprüche, **dadurch gekennzeichnet, dass** ihre Injektionsnadel (20) vorbefestigt ist und ihre Kappe (36) vor der Rekonstituierung des Präparats durch einen Blockiermechanismus (37) blockiert ist.

13. Verfahren zur Verpackung unter Vakuum im Inneren einer Karpule, die einen proximalen Behälter (13) bildet, der auf einer Seite durch einen Injektionskolben (29), der in seiner Bewegung in dem proximalen Behälter (13) blockiert ist, geschlossen ist, wobei die zu verpackenden nicht flüssigen Teile (14) in das Innere des proximalen Behälters (13) auf der Seite des Injektionskolbens (29) eingeführt werden und eine Lyophilisierung vorgenommen wird, bevor der proximale Behälter (13) durch den Kolben (23) geschlossen wird, wobei dieser Behälter (13) auf der anderen Seite zuvor geschlossen wird.

14. Verfahren nach Anspruch 13, **dadurch gekennzeichnet, dass** der Kolben (29) dann in dem proximalen Behälter (13) durch den Umgebungsdruck endgültig positioniert wird, was auf diese Weise eine Verpackungskontrolle gestattet.

15. Verfahren nach Anspruch 13 oder 14, **dadurch gekennzeichnet, dass** die zu verpackenden nicht flüssigen Teile (14) getrennt als Schicht in den proximalen Behälter (13) vor der Lyophilisierung eingeführt und eingefroren werden.

16. Verfahren nach einem der Ansprüche 13 bis 15, **dadurch gekennzeichnet, dass**
- der proximale Behälter (13) zuvor in einen Halter (19) eingeführt wird, auf dem der Kolben (29) zuvor positioniert wird, wodurch auf diese Weise eine Öffnung des proximalen Behälters (13) durch diesen Halter (19) hindurch nach außen gestattet wird; und
- dieser Kolben (29) dann (13) durch Druck und durch Bewegung in dem Halter (19), der zu seiner Führung dient, in den proximalen Behälter eingeführt wird.

17. Verfahren nach einem der Ansprüche 13 bis 16, **dadurch gekennzeichnet, dass**
- der proximale Behälter (13) zuvor in einen Halter (19) eingeführt und der Kolben (29) in einem anderen Halter (19) blockiert wird, der so eine Öffnung des Behälters zwischen den beiden Haltern (19) nach außen gestattet; und
- der Kolben (29) dann in den proximalen Behälter (13) durch Druck und durch Annäherung der beiden Halter (19) eingeführt wird.

18. Verfahren nach einem der Ansprüche 16 oder 17, **dadurch gekennzeichnet, dass** die Halter (19) dann einen Teil einer Injektionsspritzenvorrichtung und ihrer Verpakkung bilden.

19. Verfahren nach einem der Ansprüche 13 bis 18, **dadurch gekennzeichnet, dass** die zu verpackenden nicht flüssigen Teile (14) eine Mischung sind, die zuvor in dem Behälter sehr kräftig geschüttelt wird.

20. Verfahren nach einem der Ansprüche 13 bis 19, **dadurch gekennzeichnet, dass** der zylindrische Behälter zuvor in einen Halter (19) eingeführt und der Kolben (29) in einem anderen Halter (19) blockiert wird, der so eine Öffnung des Behälters nach außen zwischen den beiden Haltern (19) gestattet, wobei der Kolben dann durch Druck und durch Annäherung der beiden Halter (19) in den Behälter eingeführt wird.

21. Verfahren nach Anspruch 20, **dadurch gekennzeichnet, dass** die Halter (19) außerdem nach Annäherung luftdicht geschlossen sind und die Verpackung des proximalen Behälters (13) bilden.
